# EUROPEAN PATENT APPLICATION

(11) **EP 4 524 242 A1**
(43) Date of publication of application: **19.03.2025**
(21) Application number: 23889219.4
(22) Date of filing: 10.11.2023
(51) Int. Cl.: C12N 5/0793

(54) **METHOD FOR DIFFERENTIATING VISCEROSENSORY NEURONS FROM HUMAN INDUCED PLURIPOTENT STEM CELLS**

(30) Priority: 11.11.2022 KR 20220150261; 31.10.2023 KR 20230147745
(71) Applicant: Seoul National University R&DB Foundation, Seoul 08826 (KR)
(72) Inventor: AHN, Kyusik, Seoul 03080 (KR); MOOK-JUNG, Inhee, Seoul 03082 (KR)
(74) Representative: Staeger & Sperling Partnerschaftsgesellschaft mbB
(86) International application number: PCT/KR2023/018103
(87) International publication number: WO 2024/101963

(57) **Abstract**

The present invention relates to a method for directly differentiating Visceral sensory neurons from human-derived induced pluripotent stem cells or a composition for inducing differentiation of Visceral sensory neurons from induced pluripotent stem cells. The present invention relates to a novel protocol that can differentiate induced-Visceral sensory neurons from induced pluripotent stem cells, whereby human-derived Visceral sensory neurons can be reproduced in an *in vitro* environment. The induced-Visceral sensory neurons differentiated by the method of the present invention can be used in various fields such as drug screening utilizing the gut-neuron- brain axis, and disease models, and can be used to elucidate the pathogenesis of various sporadic neurodegenerative diseases with unclear etiology.

## Description

### TECHNICAL FIELD

The present invention relates to a method for directly differentiating Visceral sensory neurons from human-derived induced pluripotent stem cells or a composition for inducing differentiation of Visceral sensory neurons from induced pluripotent stem cells.

### BACKGROUND ART

Recently, many studies have been conducted to understand neurological diseases through the gut-brain axis. It is known that the intestinal flora changes in patients with various neurological diseases, and it has been reported that the adjustment of the intestinal flora reduces pathological findings in a mouse model of neurological disease. Many clinical trials are currently being conducted to treat various neurological diseases using the gut-brain axis, and most of these studies aim to influence the gut-brain axis by regulating the immune system, that is, the gut-blood-brain axis. However, the vagus nerve, which physically connects the gut and the brain, also plays an equally important role as the immune system in understanding the gut-brain axis.

Many clinical studies use vagus nerve stimulation techniques to treat various neurological diseases such as Alzheimer's disease. In the case of Alzheimer's and Parkinson's diseases, animal experiments have shown that the vagus nerve can deliver amyloid beta, pathological tau protein, and alpha-synuclein from the gut to the brain. These results suggest that the Gut-nerve-brain axis may play a role in delivering pathological proteins occurring in the gut to the brain and implanting them in the brain, and it is expected to be helpful in elucidating the pathogenesis of various sporadic neurodegenerative diseases with unclear etiology to date.

However, to date, only animal models exist as preclinical models to study the Gut-nerve-brain axis, and there are no *in vitro* experiments based on human cells. This is because in order to conduct *in vitro* experiments on the Gut-nerve-brain axis, the vagus nerve, which can connect the gut and the brain, must be created based on human cells. In recent studies, there have been introduced protocols that can induce colon organoids, brain organoids, and vagal motor neurons using induced pluripotent stem cells, but there is no protocol that can induce vagal sensory neurons *(i.e.,* Visceral sensory neurons) using induced pluripotent stem cells. Existing protocols for deriving sensory neurons from induced pluripotent stem cells are all Somatic sensory neurons. In the case of Somatic sensory neurons, it is known that existing sequencing data shows a clear difference in protein expression distribution compared to Visceral sensory neurons. In particular, Visceral sensory neurons have chemical receptors that can detect various neural proteins secreted by enteroendocrine cells, but Somatic sensory neurons do not have such chemical receptors. Therefore, previously reported protocols for inducing sensory neurons are not suitable for reproducing the gut- neuron-brain axis model.

### DISCLOSURE OF THE INVENTION

### TECHNICAL PROBLEM

Accordingly, the present inventors proposed a new protocol which can differentiate induced-Visceral sensory neurons from induced pluripotent stem cells.

An object of the present invention is to provide a method for differentiating Visceral sensory neurons from induced pluripotent stem cells, which includes (a) inducing an otic-epibranchial progenitor domain from induced pluripotent stem cells; (b) culturing the otic-epibranchial progenitor domain to induce epibranchial placode; and (c) differentiating the epibranchial placode into Visceral sensory neurons.

Another object of the present invention is to provide a composition for differentiating Visceral sensory neurons from induced pluripotent stem cells, including FGF-3, FGF-10, BMP-4, and IWR-1.

The present invention demonstrated the induced-Visceral sensory neurons provided according to the method above in various ways and demonstrated the reproducibility of the method. Accordingly, the induced-Visceral sensory neurons according to the method of the present invention can be used in various fields such as drug screening and disease models utilizing the Gut-nerve-brain axis.

### TECHNICAL SOLUTION

This may be explained in detail as follows. Meanwhile, each description and embodiment disclosed in the present invention may also be applied to each of other descriptions and embodiments. That is, all combinations of the various elements disclosed in the present invention pertain to the scope of the present invention. Additionally, the scope of the present invention should not be considered to be limited by the specific description described below.

An aspect of the present invention provides a method for differentiating Visceral sensory neurons from induced pluripotent stem cells.

In the present invention, "stem cell" refers to a cell, which has pluripotency to be differentiated into a cell derived from the endoderm, mesoderm, and ectoderm of an animal, or multipotency to be differentiated a cell closely related in terms of tissue or function. Additionally, the stem cell, which is an undifferentiated cell in a stage before differentiation into each cell constituting the tissue, collectively refers to cells that have the ability to be differentiated into specific cells under a specific differentiation stimulus (environment). Stem cells, unlike differentiated cells whose cell division has stopped, can produce cells identical to themselves through cell division (self-renewal), and they are characterized by having the flexibility of differentiation such that, upon application of a differentiation stimulus thereon, they can be differentiated into various cells depending on the nature of the stimulus.

These stem cells can be classified in a variety of ways, and among them, "induced pluripotent stem cells (iPSCs)" or induced pluripotent stem cells of the present invention refer to cells that have been converted into cells in an embryonic state at an early stage of development by applying a genetic mutation to somatic cells in which differentiation has been completed. The induced pluripotent stem cells can proliferate indefinitely *in vitro* and have the property to be differentiated into desired somatic cells under specific culture conditions.

The induced pluripotent stem cells of the present invention may be those prepared from somatic cells of an individual, and preferably human-derived induced pluripotent stem cells, but are not limited thereto. The human induced pluripotent stem cells may be produced by expressing reprogramming-related genes (e.g., Oct4, Sox2, Klf4, and c-Myc) in somatic cells (e.g., human fibroblasts and PBMC). In these cases, expression of Oct4, Sox2, Klf4, and c-Myc genes may be derived through retrovirus infection or the episomal system.

The term "differentiation" in the present invention refers to the phenomenon in which cells are specialized in structure or function while they grow through cell division and proliferation, that is, the shape or function of biological cells, tissues, etc. change to perform the respective assigned task.

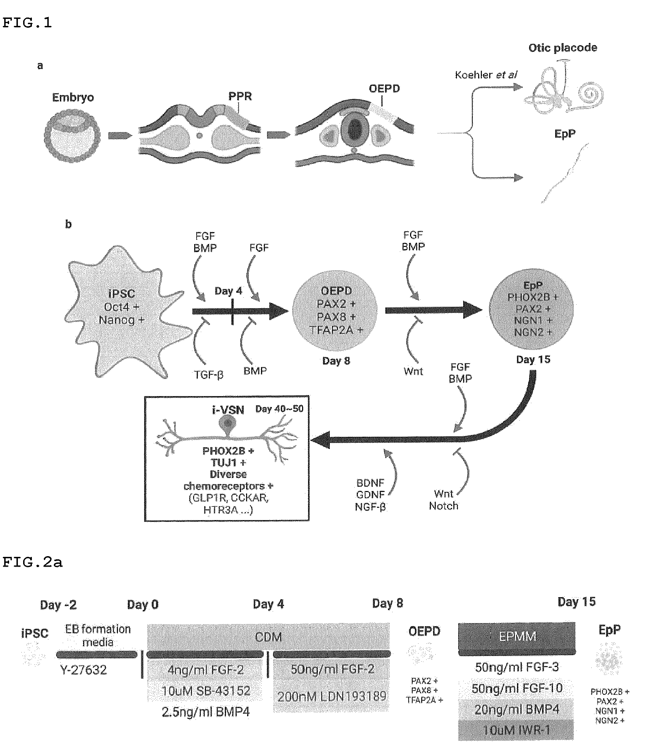

Developmentally, a non-neural ectoderm is differentiated into otic-epibranchial progenitor domain (OEPD), and the otic-epibranchial progenitor domain is differentiated into epibranchial placode (EpP) and otic placode. Visceral sensory neurons are differentiated from the epibranchial placode (FIG. 1a).

The "Visceral sensory neuron" of the present invention is a neuron that transmits the sensation in the gut to the brain, and becomes the key to reproducing the gut-brain axis in an *in vitro* environment. In view of some of the previous studies reported in which the gut-brain axis was implemented within a microfluidic system, but the limitation in existing studies was that only immune cells were used as a means of connecting the gut-brain axis. Therefore, it was possible to confirm only what effects are exhibited by the hormones secreted in the gut on the brain through blood vessels. However, it was found through many studies that the vagus nerve also plays an important role in the gut-brain axis. Accordingly, in order to establish an accurate screening platform for the gut-brain axis, it is necessary that the parasympathetic nerve *(i.e.,* the descending nerve of the vagus nerve) and the viscerosensory nerve *(i.e.,* the ascending nerve of the vagus nerve) be prepared. However, to date, only the protocols for preparing somatic sensory neurons that transmit sensations from the arms, legs, etc. to the brain have been reported, and it has been impossible to reproduce human-derived Visceral sensory neurons in an *in vitro* environment.

Meanwhile, in a previous study, a protocol for inducing the otic-epibranchial progenitor domain from induced pluripotent stem cells was disclosed (see Koehler, KR et al. Nat Biotechnol 35, 583-589 (2017)). Accordingly, in the present invention, a novel protocol was developed, in which the step of (a) inducing an otic-epibranchial progenitor domain from induced pluripotent stem cells was performed in accordance with the previous research protocol, followed by the step of differentiating the Visceral sensory neurons as a subsequent step (FIG. 1b).

Specifically, the method of differentiating Visceral sensory neurons from induced pluripotent stem cells of the present invention includes the step of inducing epibranchial placode by culturing the otic-epibranchial progenitor domain induced according to the protocol above. More specifically, the culture may be performed through a fibroblast growth factor, a bone morphogenetic protein, and a Wnt inhibitor, preferably in the presence of fibroblast growth factor-3 (FGF-3), fibroblast growth factor-10 (FGF-10), bone morphogenetic protein 4 (BMP-4), and Wnt inhibitor 1 (IWR-1), and more preferably, by mixing FGF-3, FGF-10, BMP-4, and IWR-1 in the epibranchial placode maturation medium (EPMM), but is not limited to thereto. The epibranchial placode derived through the steps above may be in the form of a pseudo-embryoid body.

The concentration of FGF-3 may be 0.1 ng/mL to 1,000 ng/mL, preferably 1 ng/mL to 100 ng/mL, more preferably 30 ng/mL to 70 ng/mL, and most preferably 50 ng/mL, but is not limited thereto.

The concentration of FGF-10 may be 0.1 ng/mL to 1,000 ng/mL, preferably 1 ng/mL to 100 ng/mL, more preferably 30 to 70 ng/mL, and most preferably 50 ng/mL, but is not limited thereto.

The concentration of BMP4 may be 0.1 ng/mL to 1,000 ng/mL, preferably 1 ng/mL to 100 ng/mL, more preferably 1 ng/mL to 50 ng/mL, and most preferably 20 ng/mL, but is not limited thereto.

The concentration of IWR-1 may be 0.1 µM to 1,000 µM, preferably 0.1 µM to 100 µM, more preferably 1 µM to 30 µM, and most preferably 10 µM, but is not limited thereto.

Additionally, the culture for inducing the epibranchial placode may be performed for 3 to 15 days, preferably 5 to 10 days, and more preferably 7 days, but is not limited thereto.

The method of differentiating Visceral sensory neurons from induced pluripotent stem cells of the present invention includes the step of differentiating the epibranchial placode induced through the steps above into Visceral sensory neurons. Specifically, the differentiation may be performed through a fibroblast growth factor, a bone morphogenetic protein, a Wnt inhibitor, a Notch inhibitor, and various nerve growth factors, preferably in the presence of FGF-3, FGF-10, BMP-4, IWR-1, DAPT (a Notch inhibitor), a brain-derived neurotrophic factor (BDNF), a glial cell-derived neurotrophic factor (GDNF), and a β-nerve growth factor (NGF-β), and more preferably, by disassembling the epibranchial placode induced in the form of a pseudo-embryoid body and culturing into a single layer, but is not limited thereto.

The culture for differentiation into the Visceral sensory neurons may be performed for 5 to 100 days, preferably 10 to 70 days, more preferably 30 to 50 days, and most preferably 40 to 50 days, but is not limited to thereto.

In the present invention, the term "Visceral sensory neuron" may be used interchangeably with "viscerosensory nerve cell". Additionally, the Visceral sensory neurons differentiated from induced pluripotent stem cells according to the method of the present invention may be referred to as "induced-Visceral sensory neurons", but is not limited thereto.

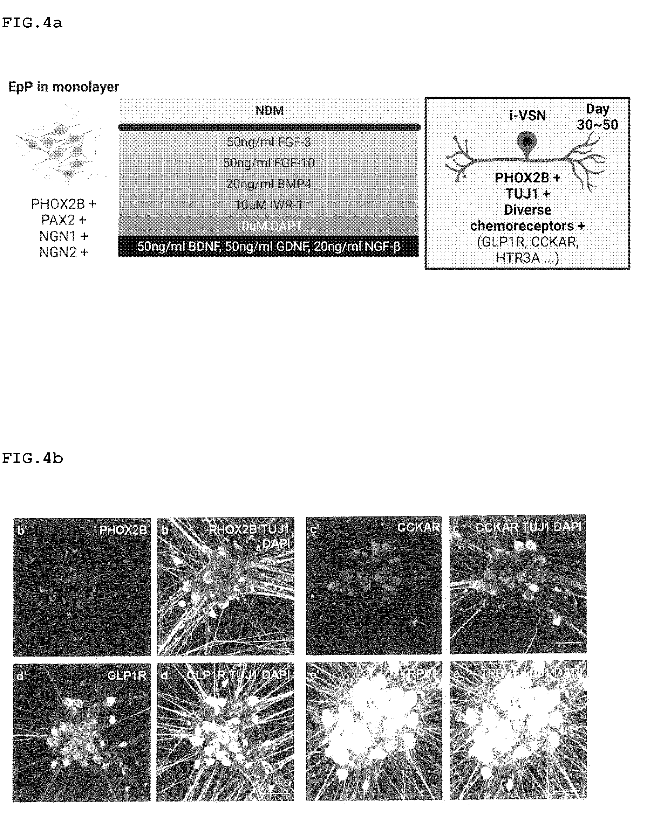

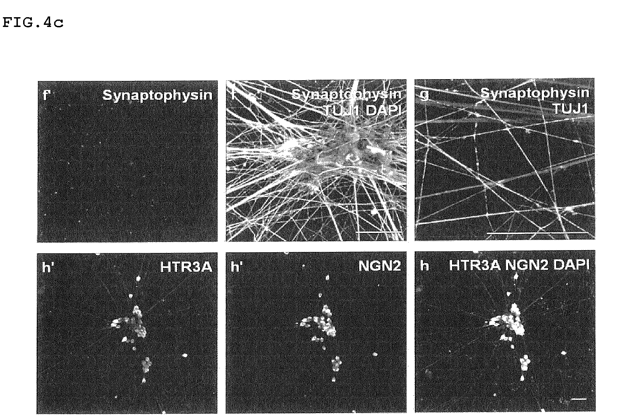

Meanwhile, in the case of the nodose ganglion of the vagus nerve, one of the representative Visceral sensory neurons, which is known to express various chemical-mechanical receptors (see Kupari, J. et al. Cell Rep 27, 2508-2523.e2504 (2019)), it was confirmed whether the Visceral sensory neurons differentiated according to the method of the present invention express various receptors such as the nodose ganglion (FIG. 4). As a result, it was confirmed that pairedlike homeobox 2b (PHOX2B) and neurogenin-2 (NGN2), which are known to be representatively expressed in the nodose ganglion; cholecystokinin A receptor (CCKAR), glucagon-like peptide-1 receptor (GLP1R), and 5-hydroxytryptamine receptor 3A (HTR3A), which are chemical receptors expressed in the nodose ganglion; and transient receptor potential vanilloid 1 (nociceptor TRPV1), which is a nociceptor, are also expressed in the Visceral sensory neurons differentiated according to the method of the present invention. Additionally, it was confirmed that synaptophysin, which is a presynaptic protein, is also expressed in Visceral sensory neurons differentiated according to the method of the present invention.

Considering the results above, the viscerosensory nerve differentiated from induced pluripotent stem cells according to the method of the present invention may be characterized in that one or more factors selected from the group consisting of PHOX2B, NGN2, CCKAR, GLP1R, HTR3A, TRPV1, synaptophysin, SSTR1, and MAP2 are expressed.

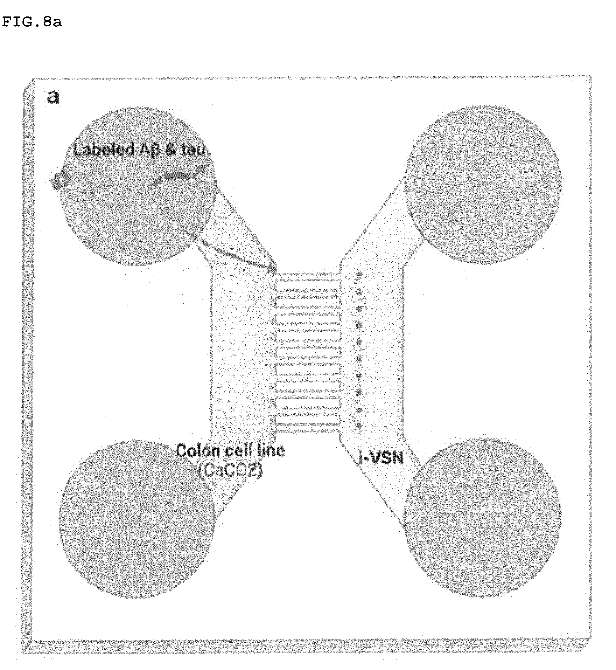

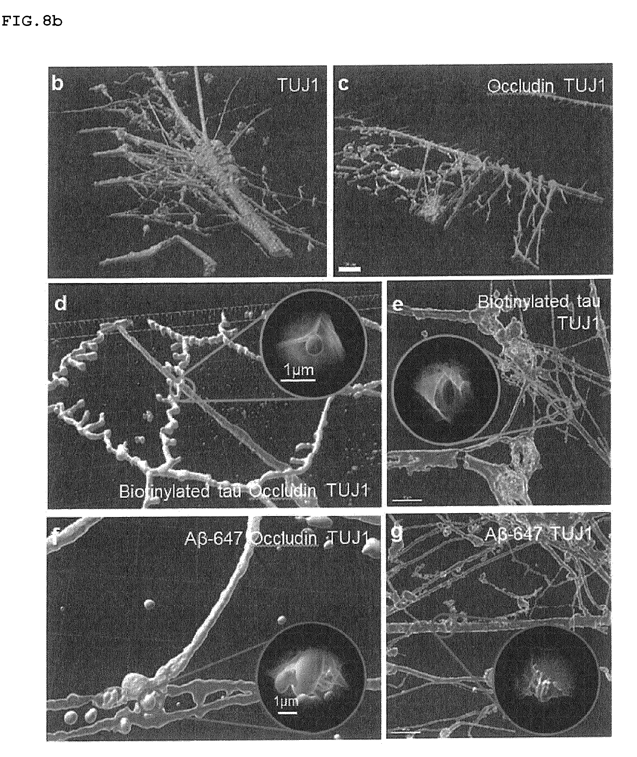

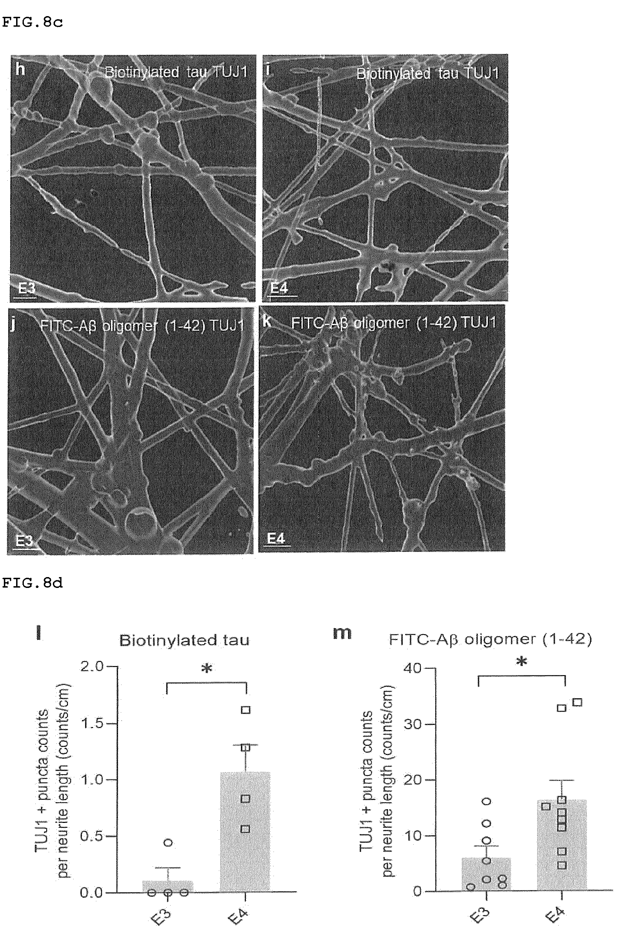

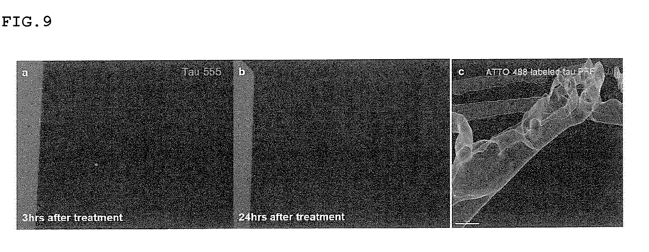

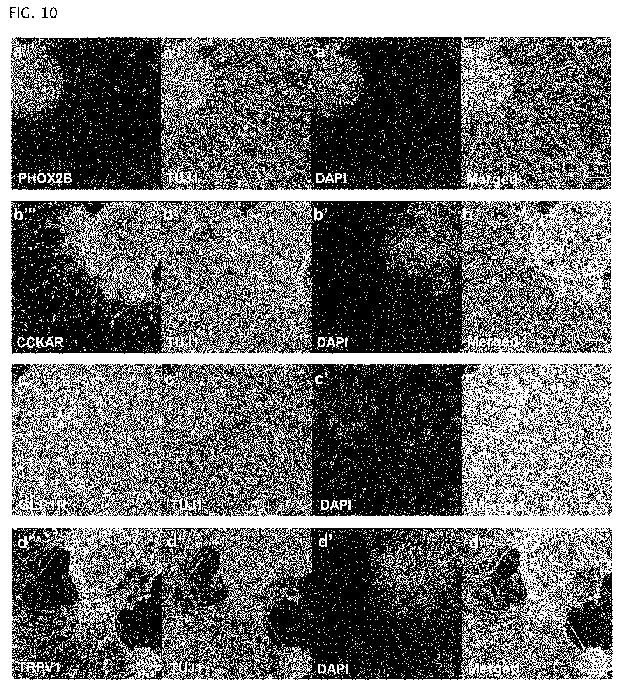

Meanwhile, it was reported in existing animal model studies that the vagus nerve can deliver amyloid beta and pathogenic tau proteins from the gut to the brain (see Chen, C. et al. Embo j 40, e106320 (2021)). However, there has been no studies as to which specific nerve between the viscerosensory nerve component and the visceral motor nerve component of the vagus nerve can play this role as a deliverer; therefore, the present inventors confirmed whether Visceral sensory neurons can act as a deliverer of pathological proteins using a microfluidic device (FIGS. 8 and 9). As a result, it was confirmed that the Visceral sensory neurons differentiated according to the method of the present invention within the microfluidic chip environment reached the colon cancer cells existing in the gut chamber, and that the Visceral sensory neurons were significantly involved in the propagation of amyloid beta and tau protein.

Considering the results above, Visceral sensory neurons differentiated from induced pluripotent stem cells according to the method of the present invention may be characterized by delivering amyloid beta or tau protein from the gut to the brain.

The "microfluidic chip" or "microfluidic device" of the present invention may be prepared using suitable methods known in the art (see Kim, C. et al. PLoS One 7, e42983 (2012)).

Another aspect of the present invention provides a composition for inducing the differentiation of Visceral sensory neurons from induced pluripotent stem cells.

The composition may further include FGF-3, FGF-10, BMP-4, and IWR-1, and may further include DAPT, BDNF, GDNF, and NGF-β.

The concentration of FGF-3 may be 0.1 ng/mL to 1,000 ng/mL, preferably 1 ng/mL to 100 ng/mL, more preferably 30 ng/mL to 70 ng/mL, and most preferably 50 ng/mL, but is not limited thereto.

The concentration of FGF-10 may be 0.1 ng/mL to 1,000 ng/mL, preferably 1 ng/mL to 100 ng/mL, more preferably 30 ng/mL to 70 ng/mL, and most preferably 50 ng/mL, but is not limited thereto.

The concentration of BMP4 may be 0.1 ng/mL to 1,000 ng/mL, preferably 1 ng/mL to 100 ng/mL, more preferably 1 ng/mL to 50 ng/mL, and most preferably 20 ng/mL, but is not limited thereto.

The concentration of IWR-1 may be 0.1 µM to 1,000 µM, preferably 0.1 µM to 100 µM, more preferably 1 µM to 30 µM, and most preferably 10 µM, but is not limited thereto.

The concentration of DAPT may be 0.1 µM to 1,000 µM, preferably 0.1 µM to 100 µM, more preferably 1 µM to 30 µM, and most preferably 10 µM, but is not limited thereto.

The concentration of BDNF may be 0.1 ng/mL to 1,000 ng/mL, preferably 1 ng/mL to 100 ng/mL, more preferably 30 ng/mL to 70 ng/mL, and most preferably 50 ng/mL, but is not limited thereto.

The concentration of GDNF may be 0.1 ng/mL to 1,000 ng/mL, preferably 1 ng/mL to 100 ng/mL, more preferably 30 ng/mL to 70 ng/mL, and most preferably 50 ng/mL, but is not limited thereto.

The concentration of NGF-β may be 0.1 ng/mL to 1,000 ng/mL, preferably 1 ng/mL to 100 ng/mL, more preferably 1 ng/mL to 50 ng/mL, and most preferably 20 ng/mL, but is not limited thereto.

Additionally, the composition for inducing differentiation of Visceral sensory neurons of the present invention may include not only essential elements for cell growth, proliferation, etc., such as sugars, amino acids, various nutrients, serum, growth factors, and minerals, but also ingredients known in the art for the culture or differentiation of cells such as stem cells.

### ADVANTAGEOUS EFFECTS

The present invention relates to a novel protocol that can differentiate induced Visceral sensory neurons from induced pluripotent stem cells, and can reproduce human-derived Visceral sensory neurons in an *in vitro* environment. The induced-Visceral sensory neurons differentiated by the method of the present invention can be used in various fields such as drug screening and disease models using the Gut-nerve-brain axis, and can be used to elucidate the pathogenesis of various sporadic neurodegenerative diseases with unknown etiology.

### BRIEF DESCRIPTION OF THE DRAWINGS

FIG. 1 is a schematic overview of differentiation of induced-visceral neurons (i-VSN). Specifically, FIG. 1a schematically shows the development process of the nodose ganglion and FIG. 1b shows the overall protocol for the differentiation of induced-Visceral sensory neurons.

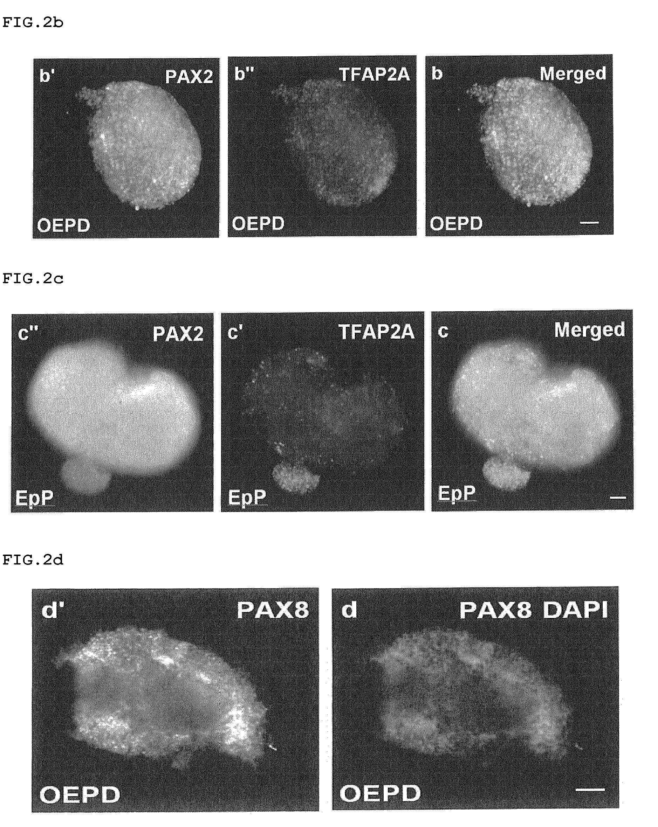

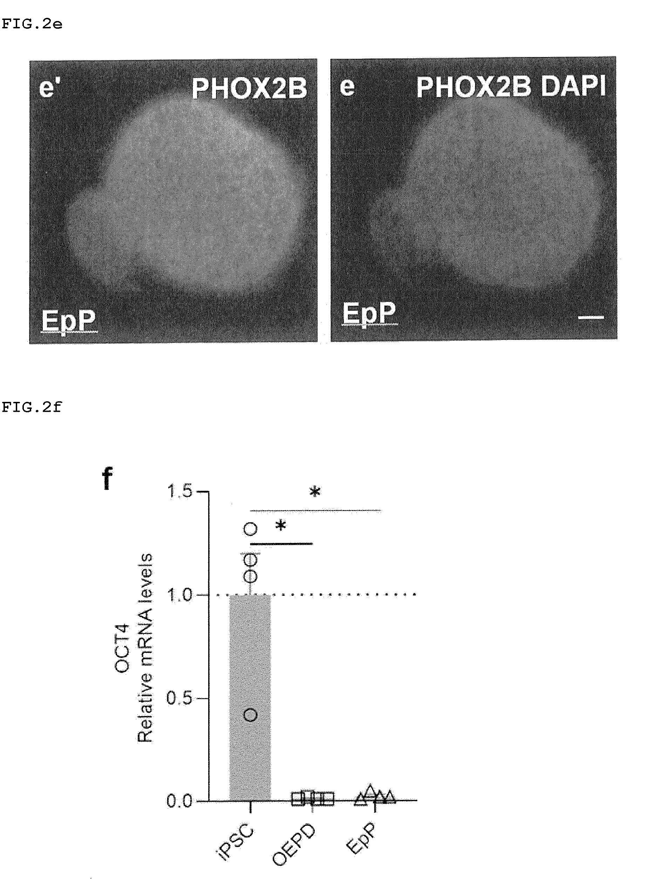

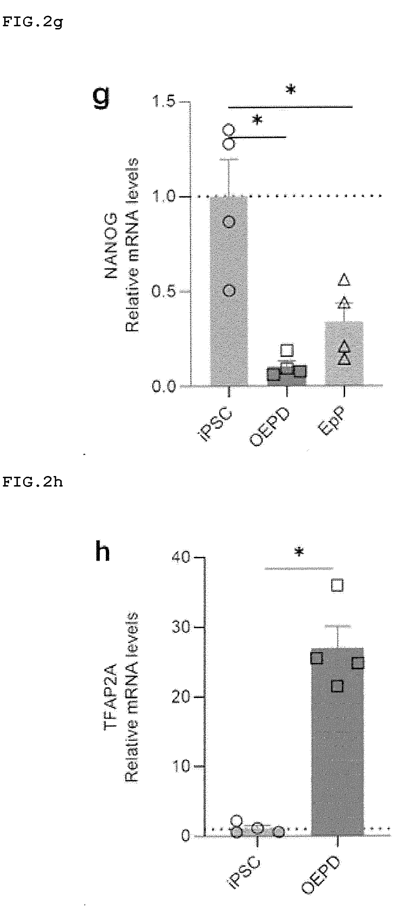

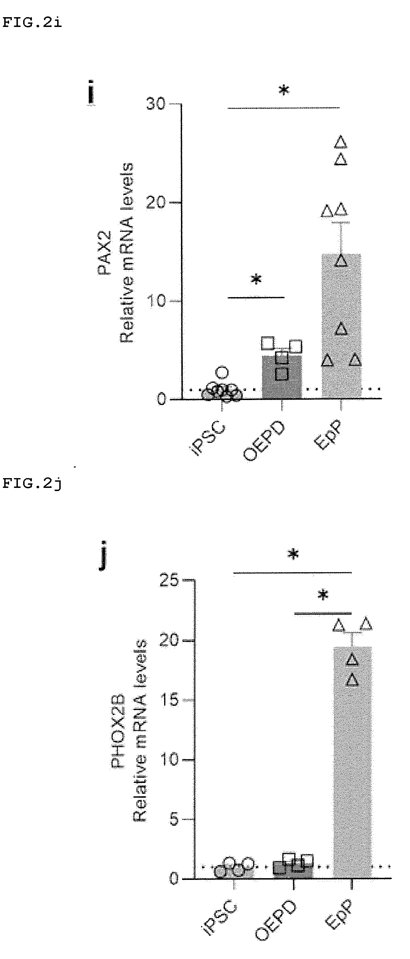

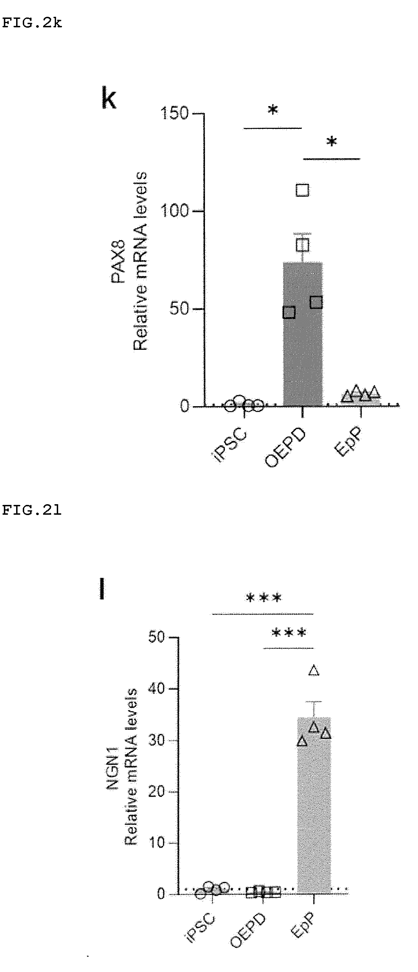

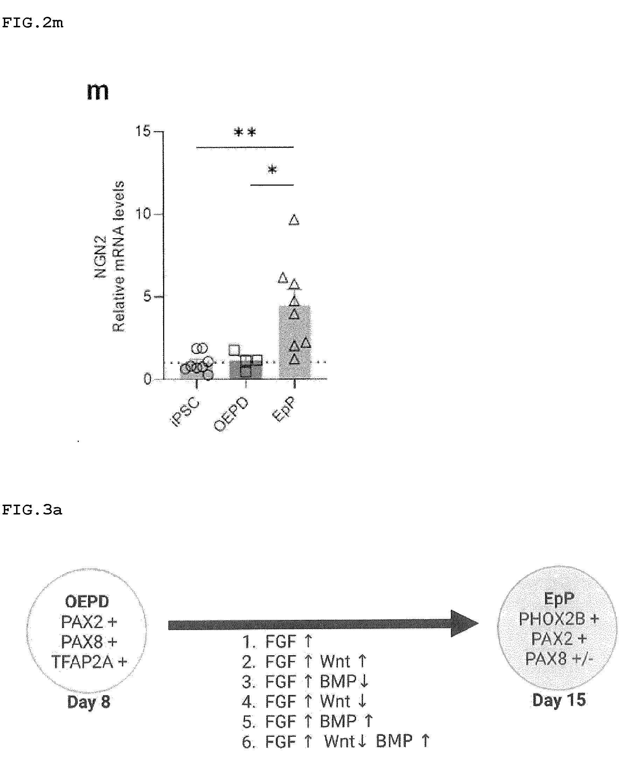

FIG. 2 shows the results confirming that epibranchial placode (EpP) was successfully differentiated from induced pluripotent stem cells (iPSCs). Specifically, FIG. 2a is a schematic diagram of the EpP differentiation protocol; FIGS. 2b and 2c show immunostaining results for PAX2 and TFAP2A in OEPD (b) and EpP (c); FIG. 2d shows immunostaining results of PAX8 in OEPD; FIG. 2e shows immunostaining results of PHOX2B in EpP; and FIGS. 2f to 2m show the RT-qPCR results comparing the relative mRNA expression of OCT4 (f), NANOG (g), TFAP2A (h), PAX2 (i), PHOX2B (j), PAX8 (k), NGN1 (1), and NGN2 (m) in iPSCs, OEPD, and EpP.

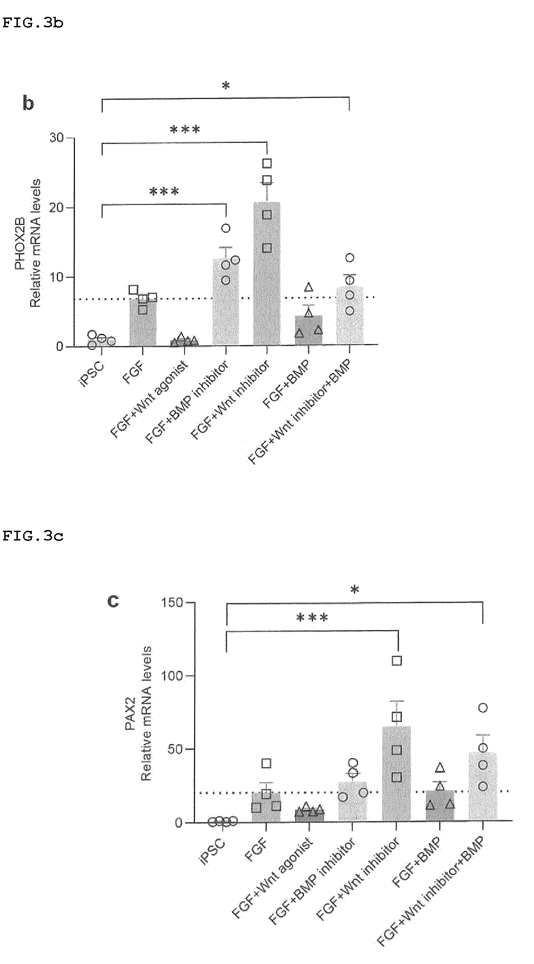

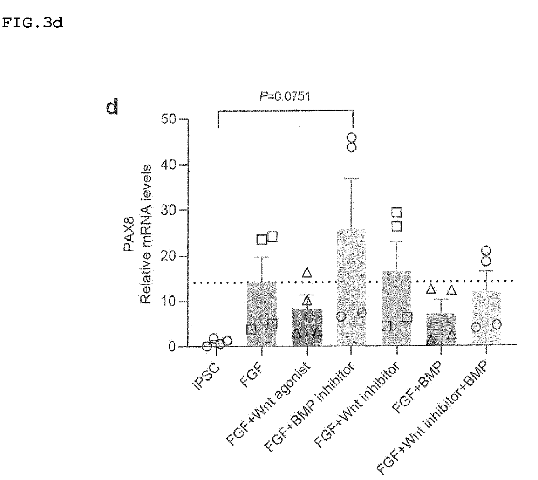

FIG. 3 shows the results of comparing various methods for inducing the epibranchial placode (EpP) from the otic-epibranchial progenitor domain (OEPD). Specifically, FIG. 3a shows various protocols tested to induce EpP from OEPD, and FIGS. 3b to 3d show the RT-qPCR results confirming the expression of PHOX2B (b), PAX2 (c), and PAX8 (d) in each protocol.
FIG. 4 shows the results of confirming standard markers of Visceral sensory neurons in induced-Visceral sensory neurons (i-VSN) differentiated from epibranchial placode (EpP). Specifically, FIG. 4a is a schematic diagram of the i-VSN differentiation protocol, and FIGS. 4b to 4h are the results of immunostaining of i-VSN.

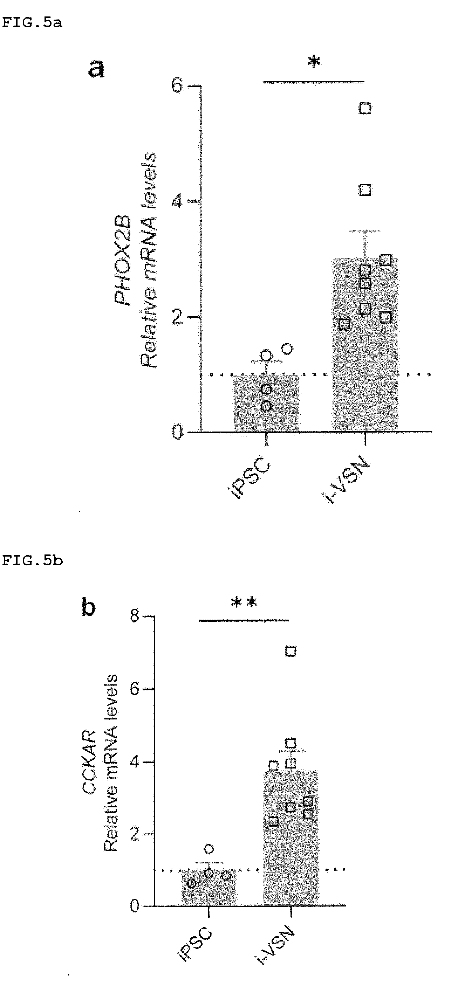

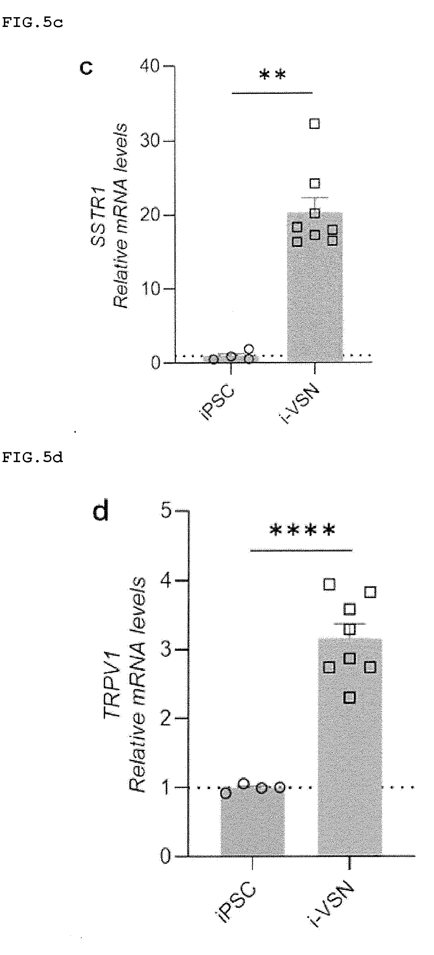

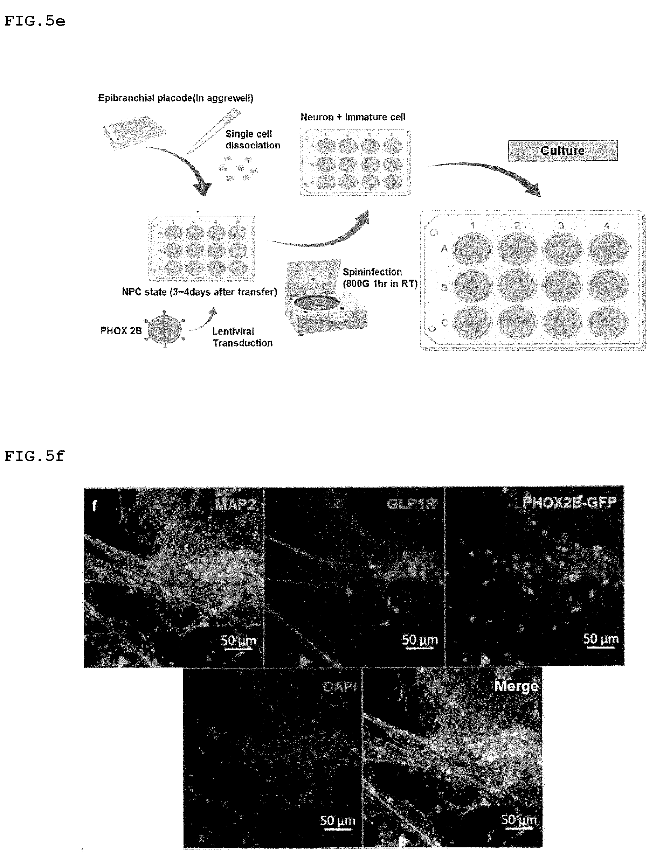

FIG. 5 shows RT-qPCR results for induced-Visceral sensory neurons (i-VSN) compared to induced pluripotent stem cells (iPSCs). Specifically, FIGS. 5a to 5d show the expression levels of PHOX2B, CCKAR, SSTR1, and TRPV1 in i-VSN; FIG. 5e is a schematic diagram of the protocol for the transduction of induced-Visceral sensory neurons using lentivirus including PHOX2B-GFP; and FIG. 5f shows the results of immunostaining for MAP2 (Orange), which is a neural marker, GLP1R (Red), one of the chemical receptors, and GFP signal (Green), and DAPI, after transduction.

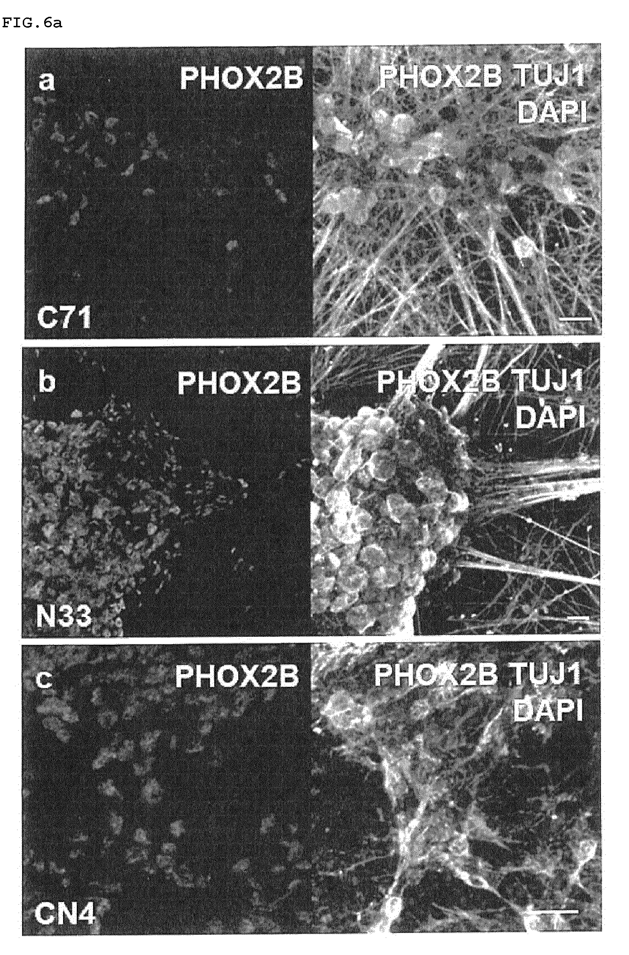

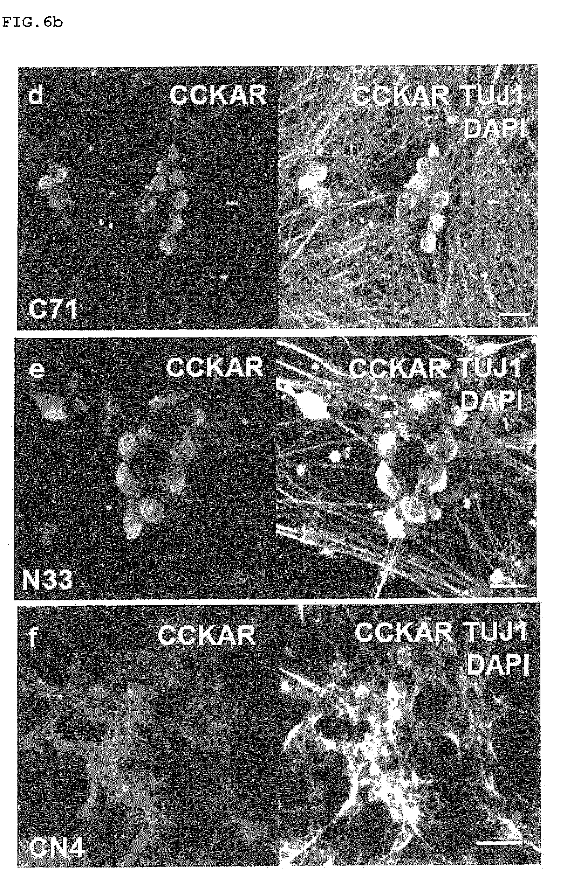

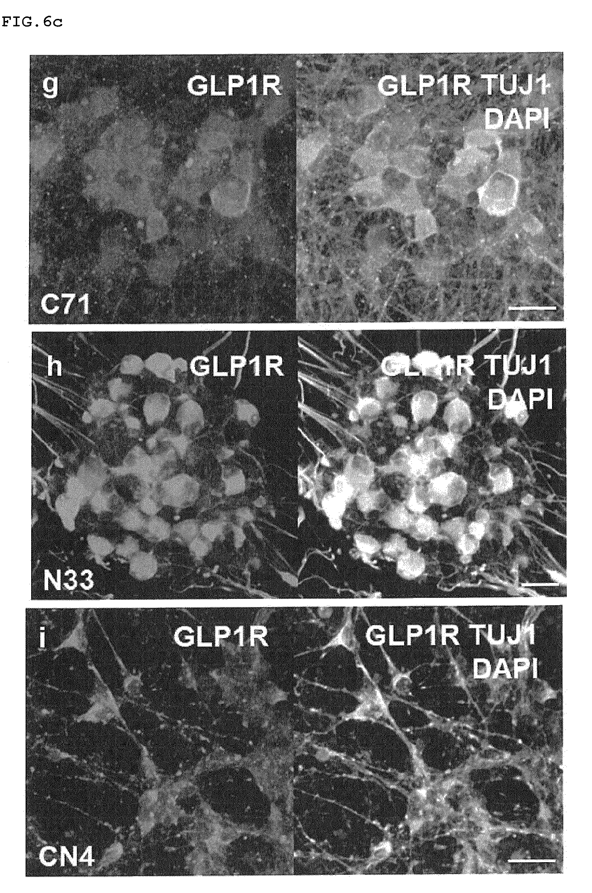

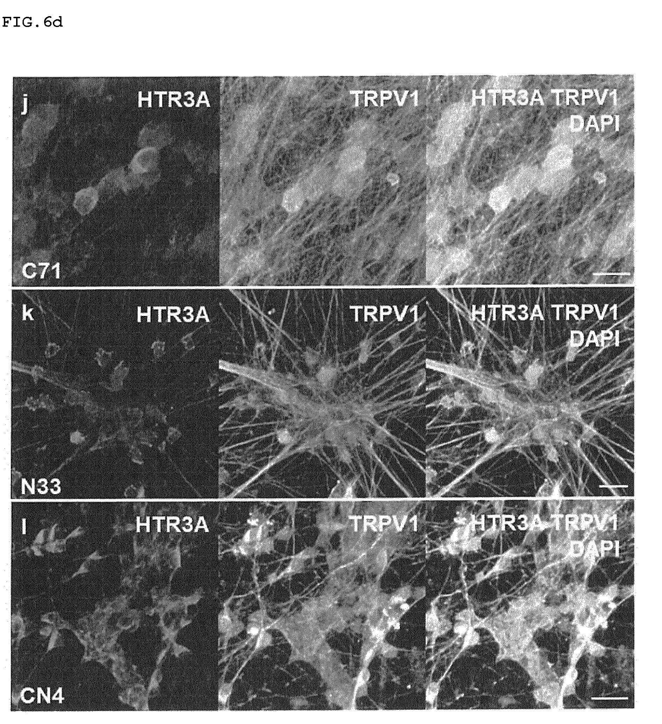

FIG. 6 shows the results confirming that the differentiation protocol for induced-Visceral sensory neurons(i-VSN) of the present invention is reproducible in several pluripotent stem cell (iPSC) lines.

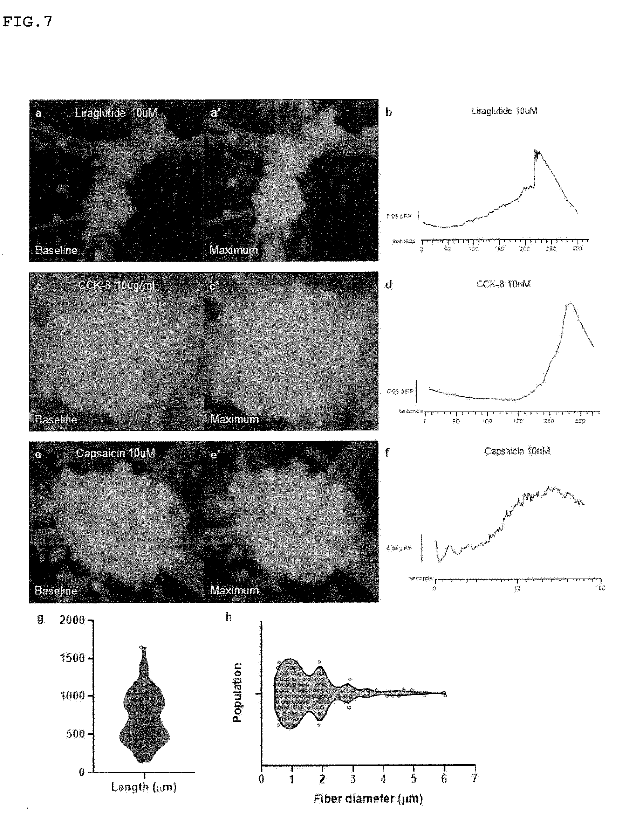

FIG. 7 relates to the functional and external characteristics of the induced-Visceral sensory neurons (i-VSN). Specifically, FIGS. 7a to 7f show functional calcium images of i-VSN after the treatment with various compounds, and FIGS. 7g and 7h show the distribution of the nerve fiber length and diameter of cultured i-VSN.
FIG. 8 shows the results of confirming the proliferation analysis of beta-amyloid and tau through a microfluidic chip connecting induced-Visceral sensory neurons (i-VSN) and Caco-2 cells. Specifically, FIG. 8a is a schematic diagram of the microfluidic device connecting i-VSN (neuron chamber) and Caco-2 (gut chamber); FIG. 8b is a rendered image of a neuron chamber; FIG. 8c is a rendered image of the gut chamber; 8d to 8g are rendered images of radio wave analysis; and FIGS. 8h to 8m show the results of quantifying the propagation analysis of biotinylated tau or FITC-labeled beta-amyloid oligomers.
FIG. 9 shows the results of analysis of proliferation of beta-amyloid and tau in induced-Visceral sensory neurons (i-VSN) through a microfluidic chip. Specifically, FIGS. 9a and 9b show the hydrostatic barrier effect in the propagation analysis, and FIG. 9c shows the immunostaining results rendered after processing tau PFF labeled with ATTO 488.
FIG. 10 shows the results of immunostaining for induced-Visceral sensory neurons (i-VSN) neurospheres.

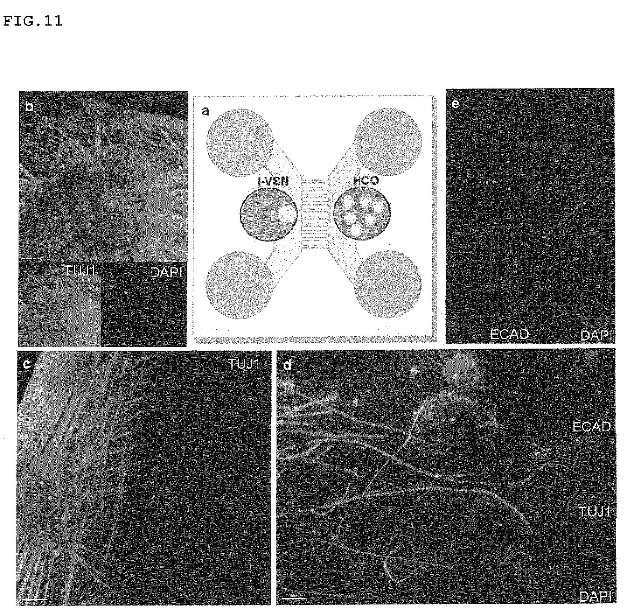

FIG. 11 shows an axis-mimetic-chip model that mimics a gut-brain axis.

### MODE FOR CARRYING OUT THE INVENTION

Hereinafter, the present invention will be described in more detail through examples. However, these examples are for illustrative purposes only and the scope of the present invention is not limited to these examples.

### Materials and Methods

### (1) Method for maintaining induced pluripotent stem cells

Human induced pluripotent stem cells were maintained using mTeSR^{™} Plus media (Stemcell technologies, 110-1130, hereinafter referred to as "mTeSR+ media"). Cells were cultured in 6-well culture plates coated with hESC-qualified Matrigel (Corning, 354277). Subculture was performed when the cells filled 80% or more of the microscope screen, and the separation agent used at this time was ReLeSR (Stemcell technologies, ST05872) and the separation was performed at a 1:20 cell ratio. In order to minimize genetic loss occurring during repeated subculture, the number of subcultures was set to 30 or less.

### (2) Method of inducing epibranchial placode (EpP) embryoid bodies

Embryoid bodies were induced in the specialized AggreWell^{™}800 24-well Plate (Stemcell technologies, 34815, hereinafter "AggreWell") to prepare embryoid bodies. Before differentiation, lubrication was performed by adding 500 µL of Anti-Adherence Rinsing Solution (Rinsing Solution, Stemcell Technologies, 07010) to each well in the AggreWell. After lubrication, 2 mL of DMEM/F-12 (Gibco, 10565018) was added to each well. In order to form embryoid bodies in AggreWell, induced pluripotent stem cells were treated with Accutase (Sigma, A6964) including Y-27632 (Stemcell Technologies, ST72304) at a concentration of 20 µM in a cell culture dish, and then the cells were carefully separated from the plate. Thereafter, the cells in each well were dissolved with 2 mL of AggreWell^{™} EB Formation Medium (Stemcell Technologies, 05893, hereinafter referred to as "EB media"), such that an equal amount of 5 × 10⁴ cells were added to each well (day 2 of culture). On day -1 of culture, the media was partially replaced (1.5 mL per well) and replaced with EB media without other supplements. On day 0 of culture, the media was partially replaced with Chemically-Defined Differentiation Medium (CDM; see previous description), supplemented with 4 ng/mL of FGF-2 (R&D Systems, 233-FB-025); supplemented with 10 µM SB-431542, and 2.5 ng/mL of BMP4 (Peprotech, AF-120-05ET). The same media replacement was also performed on day 2 of culture. On day 4 of culture, the media was partially replaced with CDM supplemented with 50 ng/mL of FGF-2 and 200 nM LDN193189 (Tocris Bioscience, 6053). The same media replacement was also performed on days 6 and 7 of culture. On day 8 of culture, epibranchial placode maturation medium (EPMM, Table 1), which was supplemented with 50 ng/mL of FGF-3 (R&D Systems, 1206-F3-025/CF), 50 ng/mL of FGF-10 (R&D Systems, 345-FG-025/CF), 20 ng/mL of BMP-4, and 10 µM IWR-1 (Sigma, I0161-5MG), was partially replaced. The same media replacement was performed every day until Day 14.

**[Table 1]**

| **Component** | **Supplier** | **Catalogn no** | **Stock conc** | **Final conc** | **Volume needed (ml)** |
|---|---|---|---|---|---|
| **Adv IMEM/F12** | Gibco | 12634010 | - | 49% (v/v) | 24.28ml |
| **Neurobasal** | Gibco | 21103-049 | - | 49% (v/v) | 24.28ml |
| **N2 supplement** | Gibco | 17502-048 | 100x | 0.5x | 250ul |
| **B27-Vitamin A** | Gibco | 12587-010 | 50x | 0.5x | 500ul |
| **GlutaMAX** | Gibco | 35050-079 | 100x | 1x | 500ul |
| **Mercaptoethanol** | Gibco | 21985-015 | 55mM | 0.1mM | 90.91ul |
| **Normocin** | Invivogen | Ant-nr-1 | 50mg/ml | 100ug/ml | 100ul |
| **Total** | | | | | 50 |

The table above relates to the epibranchial placode maturation medium (EPMM).

### (3) Method for differentiation of induced-Visceral sensory neurons

On day 15 of culture, embryoid bodies present in the AggreWell were separated into single cells using Accutase including 20 µM Y-27632. The separated cells were administered at 4.0-8.0 × 10⁵ cells per well in a 12-well culture plate coated with PLO/laminin. For the culture of induced-Visceral sensory neurons neurospheres, embryoid bodies were directly treated on plates coated with PLO/laminin or domes including Matrigel (Corning, 356231). Nodose ganglion differentiation medium (NDM, Table 2) was used as culture media, including 20 µM Y-27632, 10 µM IWR-1, 10 µM DAPT (Sigma, D5942), 20 ng/mL of BMP-4, 50 ng/mL of FGF-3, 50 ng/mL of FGF-10, 50 ng/mL of BDNF (Peprotech, 450-02), 50 ng/mL of GDNF (Peprotech, 450-10), and 20 ng/mL of NGF-β (Peprotech, 450-01), and was used 1 mL per well. On the next day, all media were replaced with the same media excluding Y-27632. Thereafter, the media was partially exchanged (500 µL in a total media of 1.5 mL) every 2 to 3 days. 2 µg/mL of laminin (Sigma, L2020) was added to the media every two to three times when the media was replaced.

The analysis was performed between days 40 and 50.

**[Table 2]**

| **Component** | **Supplier** | **Catalog no** | **Stock conc** | **Final conc** | **Volume needed** |
|---|---|---|---|---|---|
| **Neurobasal** | Gibco | 21103-049 | - | 49% (v/v) | 47.81ml |
| **N2 supplement** | Gibco | 17502-048 | 100x | 1x | 500ul |
| **B27-Vitamin A** | Gibco | 12587-010 | 50x | 1x | 1000ul |
| **GlutaMAX** | Gibco | 35050-079 | 100x | 1x | 500ul |
| **Mercaptoethanol** | Gibco | 21986-015 | 55mM | 0.1mM | 90.9ul |
| **Normocin** | Invivogen | Ant-nr-1 | 50mg/ml | 100ug/ml | 100ul |
| **Total** | | | | | 50 |

The table above relates to the nodose ganglion differentiation medium (NDM).

### (4) Transduction of Visceral sensory neurons using lentivirus including PHOX2B-GFP

In the case of the lentiviral vector including PHOX2B-GFP, the one purchased from Origene was used (Origene, RC200441L4). The vector for the corresponding virus was transformed into Stbl3, which is a type of *E. coli* that is a competent cell, to produce the vector, and as such, the corresponding vector was transfected into a HEK cell line using the Origene packaging vector (Origene, TR30037) to produce lentivirus. The produced lentivirus was treated onto the cells in epibranchial placode state cultured as single cells on the plate at a rate of about 10 MOI on day 15 of culture. Thereafter, the resultant was subjected to centrifugation in a centrifuge at room temperature at 800G for 1 hour, and transduction was performed for 24 hours, after which the media was replaced.

### (5) Method of differentiating colon organoids

Colon organoids were differentiated according to a previously described protocol (see Crespo, M. et al. Nat Med 23, 878-884 (2017)). In summary, the separated induced pluripotent stem cells were treated in hESC-qualified Matrigel-coated 6-well culture plates at a density of 2.0 × 10⁵ cells per well. Media was replaced with mTeSR+ media every two days until cells reached 80% density. When the induced pluripotent stem cells reached the corresponding density, the media was replaced with RPMI 1640 media (Gibco, 11875093), which included 2 mM GlutaMAX supplement (Gibco, 35050079), 100 ng/mL of Activin A (Cell Guidance Systems, GFH6)), 3 µM CHIR99021 (Tocris Bioscience, 4423), and 100 U/ml Pen-strep (Sigma, P4333 (day 1 of culture). On day 2 and day 3 of culture, the media was replaced daily with RPMI 1640 media supplemented with 2 mM GlutaMAX supplement, 100 ng/mL of Activin A, 0.2% BSA (Gibco, 15260037), and 100 U/mL of Pen-strep. From day 4 to day 7 of culture, the media was replaced daily with RPMI 1640 media containing 2 mM GlutaMAX supplement, 1X B27 supplement (Gibco, 12587-010), 3 µM CHIR99021, 500 µg/mL of FGF-4 (Peprotech, 110-31), and 100 U/mL of Pen-strep. On day 8 of culture and thereafter, the media was replaced daily with Advanced DMEM/F12 (Gibco, 12634010) media containing 2 mM GlutaMAX supplement, 1X B27 supplement, 3 µM CHIR99021, 100 ng/mL of EGF (Peprotech, AF-100-15), 300 nM LDN193189, and 100 U/mL of Pen-strep. On day 20 of culture and thereafter, floating spheres were carefully collected using a pipette, dispersed in Matrigel, and inserted into a 24-well culture plate or the organoid chamber of the microfluidic chip. For the 24-well culture plate, 40 µL of Matrigel was used per well, and for the microfluidic chip, 10 µL of Matrigel was used per chamber. After the insertion, the media was replaced once every two days with Advanced DMEM/F12-based media described previously. Finally, 3 days before harvest, the media was replaced with Advanced DMEM/F12-based media including growth factors previously described excluding CHIR99021.

### (6) Method for maintaining colon cancer cell lines

Caco-2 cell line was used as the colon cancer cell line. The maintenance and mucosalization of the Caco-2 cell line were performed according to previously described protocols. For the culture of caco-2 cells, 20% FBS and 100 U/mL of Pen-strep were added to DMEM media (Gibco, 10438034). The media was replaced daily, and the cells were detached for subculture using a 0.25% trypsin/1 mM ethylenediaminetetraacetic acid (EDTA) solution.

### (7) Reverse transcription quantitative PCR (RT-qPCR)

All steps were performed according to the documented manufacturer's protocol. The RNA isolation was performed using the RNeasy Plus Mini Kit (QIAGEN, 74136), cDNA synthesis was achieved using the Maxime RT PreMix kit (iNtRON BIOTECHNOLOGY, 25081), and real-time quantitative PCR (qPCR) was performed using the KAPA SYBR FAST qPCR universal kit (KAPA Biosystems, kk4602). All of the sequences of the primers used are described in Table 3 below. The expression level was confirmed using ΔΔCt of each target mRNA. L27 was used as the housekeeping gene.

**[Table 3]**

| **Name** | **Forward** | **Reverse** |
|---|---|---|
| Oct4 | GGAGGAAGCTGACAACAATGAAA | GGCCTGCACGAGGGTTT |
| Nanog | CATGAGTGTGGATCCAGCTTG | CCTGAATAAGCAGATCCATGG |
| TFAP2A | GATCCTCGCAGGGACTACAG | T4CCCGGGTCTTCTACATGC |
| PAX8 | GCCCAGTGTCAGCTCCATTA | GCTGTCCATAGGGAGGTTGAA |
| PHOX2B | CTCGGTTTGCATTTCTGTTGG | TGTCTAAACTGCGCGAAGAATG |
| PAX2 | CTTTAAGAGATGTGTCTGAGGG | CCTGTTCTGATTTGATGTGCT |
| NGN1 | GCCTCDGAAGACTTCACCTACC | GGAAAGTAACAGTGTCTACAAAGG |
| NG42 | AGGAAGAGGACGTGTTAGTGC | GCAATCGTGTACCAGACCCAG |
| L27 | CGTGAAGAACATTGATGATGGC | GCGATCTTCTTCTTGCCCAT |

### (8) Immunochemical staining

Induced-Visceral sensory neurons were cultured on PLO/laminin-coated glass slides in 12-well culture plates. For the experiment, the media was removed and rinsed three times with phosphate-buffered saline (PBS). Then, the cells were fixed with 4% paraformaldehyde at 4°C for 20 minutes. After fixation, the wells were rinsed three times with phosphate-buffered saline. The blocking and permeabilization solution (BPS) consisted of 0.3% Triton, 5% normal horse serum (Vector, S-2000-20), and 0.5 mg/mL of BSA mixed in PBS. The blocking and permeabilization solution (BPS) was treated at room temperature for 1 hour. Thereafter, the primary antibody mixed in the blocking and permeabilization solution was treated at 4°C for 16 to 24 hours. The list of major antibodies used is described in Table 4 below. Then, the wells were rinsed three times with phosphate-buffered saline, and then treated with a solution, in which 5% normal horse serum and secondary antibody were mixed in phosphate-buffered saline, and placed at room temperature for 1 hour. Thereafter, the wells were rinsed three times with phosphate-buffered saline, and treated with DAPI at room temperature for 15 minutes. Finally, the wells were rinsed three times with phosphate-buffered saline and placed on a glass slide for fluorescence microscopy imaging.

**[Table 4]**

| **Antibody name** | **Host** | **Supplier** | **Catalog number** | **Dilution** |
|---|---|---|---|---|
| PAX8 | Rabbit | Abcam | AB97477 | 1:100 |
| Ngn2 | Rabbit | Novus biologicals | NBP2-96817 | 1:300 |
| PAX2 | Rabbit | Invitrogen | 716000 | 1:100 |
| TFAP2 | Mouse | DSHB | 3B5 | 1:5 |
| HTR3A | Mouse | Invitrogen | MA5-31770 | 1:200 |
| TRPV1 | Rabbit | Novus biologicals | NB120-3487 | 1:200 |
| PHOX2B(C-T) | Rabbit | Abcam | ab183741 | 1:100 |
| GLP1R | Rabbit | Novus | NBP1-97308 | 1:100 |
| CCKAR | Rabbit | Thermo | PA3-116 | 1:250 |
| TUJ1 | Rabbit | Abcam | Ab18207 | 1:1000 |
| TUJ1 | Mouse | Abcam | Ab78078 | 1:1000 |
| Synaptophysin | Mouse | BD Transduction laboratories | 611880 | 1:1000 |

### (9) Photographing of fluorescent images and analysis thereof

Images were taken using a ZEISS LSM700 polarizing microscope and an EVOS imaging system. In order to measure the length and the diameter of nerve fibers of two-dimensional induced Visceral sensory neurons, based on the light microscopy images taken by the EVOS, the length and the diameter were manually measured for using celleste6 software (Thermo Fisher Scientific, ver 6.0). For three-dimensional reconstruction and rendering, the Imaris program (Imaris for neuroscientists, Oxford instruments, ver 10.0) was used.

For quantitative propagation experiments, fluorescence microscopy images were captured in the nerve fiber chamber (in an opposite side of the neuron chamber) and the number of amyloid or tau proteins was quantified by dividing the same by the nerve fiber length. The nerve fiber length was measured using Imaris filament analysis. In order to measure the number of uptake of amyloid beta or tau, the number of amyloid or tau which showed positive for TUJ1 signal was counted.

### (10) Live cell calcium imaging

For Live cell calcium imaging, the FLIPR Calcium 6 Assay Kit (R8290, Molecular Devices) was used. The overall steps were performed according to the manufacturer's protocol. The test solution, in which components A and B were mixed, was incubated in an incubator at 37°C for 2 hours, and during this process, the carbon dioxide concentration was maintained at 5%. Then, after treatment with a fluorescent substance, the fluorescence signal was detected for 5 minutes using an EVOS live imaging system (Thermo-Fisher) capable of maintaining a temperature of 37°C, a 5% carbon dioxide concentration, and 80% humidity. The detected signals were obtained by video or continuous snapshots (once per second).

### (11) Microfluidic device

A previously described method was used to form the microfluidic device. In summary, a PDMS polymer precursor and a catalyst (Dow Hi-Tech, Sylgard 184 A/B) were completely mixed in a 10:1 ratio and injected into a mold. In order to remove air bubbles, the PDMS-treated mold was placed in a vacuum dehydrator for 1 hour. After removing the air bubbles, the PDMS mold was cured in a drying oven at 60°C for 4 hours. After the curing step, the PDMS was separated from the mold and an aperture with a diameter of 8 mm was generated in the storage chamber. For the three-dimensional axis-mimetic-chip model, a biopsy punch with a diameter of 6 mm was used for the storage chamber, and a biopsy punch with a diameter of 4 mm was applied to the additional organoid chamber. Dust was removed with a silicone-free adhesive plastic film (Ultron Systems) before bonding the PDMS to the glass coverslip. PDMS was permanently bonded to the glass coverslip by performing plasma treatment. Thereafter, the microfluidic device was placed on a heating plate at 100°C for 3 minutes to remove remaining moisture. Finally, PLO/laminin coating was performed on the device.

In the case of the three-dimensional axis-mimetic-chip model, considering the difference in time required for complete maturation between the induced-Visceral sensory neurons and colon organoids, the dates of insertion of the induced-Visceral sensory neurons and colon organoids were set to be different. After inserting the epibranchial placode analog into the chip on day 15 of culture, the colon organoids were inserted 10 days later, which was day 15 of culture. For both induced-Visceral sensory neurons and colon organoids, one to two spheres were inserted into 10 µL of Matrigel for each microfluidic chip.

### (12) Preparation of fluorescent or biotin-labeled beta-amyloid and tau

In the cases of the tau-441 monomer labeled with HiLyte Flour 555 (Anaspec, custom-made), biotinylated tau-441 (Anaspec, AS-56084) and tau preformed fibers labeled with ATTO 488 (Stressmarq, SPR-329-A488), they were used directly in a state dissolved in 10 mM sodium phosphate buffer at a stock concentration of 1 mg/mL.

In the case of beta amyloid (1-42) labeled with HiLyte Flour 647 (Anaspec, AS-64161), 0.1 mg of the labeled beta amyloid was first dissolved in 50 µL of 1% NH₄OH solution and then diluted with 50 µL of phosphate-buffered saline to a final concentration of 1 mg/mL or 180 µM.

In the case of the FITC-labeled beta amyloid (1-42) oligomer, FITC-labeled beta amyloid (1-42) (Bachem, 4033502) was used. For monomerization, 1 mL of HFIP was treated per 1 mg of the FITC-labeled beta amyloid. After HFIP treatment, the dissolved beta-amyloid was placed on a rotating stirrer and multimerized at room temperature for 72 hours. After 72 hours, moisture was removed from the solution using a SpeedVac device (Thermo-Fisher, SPD2010-220) and then the resultant was stored in an ultra-low temperature freezer at -80°C to be lyophilized. Finally, the lyophilized amyloid was dissolved in DMSO (Sigma, D4540) and used at a density of 1 µM.

### Statistical analysis

Statistical analyses were performed using Medcalc20.113 (MedCalc Software, Ostend, Belgium) and GraphPad Prism 8 (Graphpad Software, CA, USA). The Student t-test or Tukey's *post hoc* test was used to compare parametric variables, and the Mann-Whitney U test or Conover's post hoc test was used to compare nonparametric variables.

### Examples

### Example 1: Process of differentiation from induced pluripotent stem cells to epibranchial placode

Developmentally, the non-neuralectoderm is differentiated into the otic-epibranchial progenitor domain (OEPD), and the otic-epibranchial progenitor domain (OEPD) is differentiated into an epibranchial placode (EpP) and an otic placode. Between them, Visceral sensory neurons are differentiated from the double epibranchial placode (FIG. 1a). In previous studies, a protocol for inducing from induced pluripotent stem cells to the otic-epibranchial progenitor domain was disclosed. In this regard, in the present invention, the differentiation was performed according the protocol of the previous study up to the differentiation of the otic-epibranchial progenitor domain, and a new protocol was developed for the differentiation thereafter (FIG. 1b). In animal experiments, it is known that for the differentiation of the epibranchial placode in the otic-epibranchial progenitor domain, fibroblast growth factor (FGF) and bone morphogenetic protein (BMP) signals are required, while Wnt signaling (Wnt) should be suppressed (FIG. 1b). Among various fibroblast growth factors, fibroblast growth factor 3 (FGF-3) and fibroblast growth factor 10 (FGF-10) had been known to play a key role in differentiating the epibranchial placode, and the mechanism was known to occur by amplifying the PAX2 signal of ectoderm origin. Therefore, the culture was performed by mixing fibroblast growth factor 3 (FGF-3), fibroblast growth factor 10 (FGF-10), bone morphogenetic protein 4 (BMP-4), and IWR-1, which is a Wnt inhibitor, to the epibranchial placode maturation medium (EPMM) (FIG. 2a and Table 1).

First, each developmental stage was verified by real-time quantitative PCR (RT-qPCR) and immunostaining. It was confirmed through immunofluorescence staining that PAX2, TFAP2A, and PAX8, which are known to be expressed in the otic-epibranchial progenitor domain, were all expressed at the otic-epibranchial progenitor domain stage (FIGS. 2b and 2d). Additionally, as previously identified, it was confirmed that PAX2 is expressed at the epibranchial placode stage and that TFAP2A expression is decreased (FIG. 2c). PHOX2B was also confirmed to be expressed at the epibranchial placode stage (FIG. 2e). In addition to immunostaining, in the case of RT-qPCR results, it was confirmed that the expression of OCT4 and NANOG was decreased with statistical significance after the otic-epibranchial progenitor domain stage (FIGS. 2f and 2g). Additionally, similar to the immunostaining, the expression levels of PAX2, PAX8, and TFAP2A remained high in the otic-epibranchial progenitor domain stage (FIGS. 2h, 2i, and 2k). At the epibranchial placode stage, the PAX2 expression level remained high, but the PAX8 expression level was decreased (FIGS. 2i and 2k). Compared to previous stages, the epibranchial placode was confirmed to have high expression levels of PHOX2B, NGN1, and NGN2 (FIGS. 2j, 2l, and 2m). It can be seen that these results are consistent with existing developmental studies.

In order to confirm whether the combination of growth factors previously treated is the most effective method for inducing epibranchial placode in the otic-epibranchial progenitor domain, the effects were compared through various combinations that activate or inhibit fibroblast growth factor, a Wnt inhibitor, and bone morphogenetic protein signaling pathways (FIG. 3a). As a result, it was confirmed that among various combinations, when the currently used fibroblast growth factor, bone morphogenetic protein, and Wnt inhibitor were used, the expression levels of PHOX2B and PAX2 were significantly increased, but the expression level of PAX8 was not increased (FIGS. 3b to 3d). Based on these results, it was confirmed that the method presented in the present invention is the most efficient method for inducing epibranchial placode from induced pluripotent stem cells.

### Example 2: Demonstrating that various proteins expressed by induced-Visceral sensory neurons are similar to actual Visceral sensory neurons

Next, in order to express induced-Visceral sensory neurons at the epibranchial placode stage, several growth factors were added. For neural differentiation, embryoid bodies were disassembled into cells and cultured into a single layer. Similar to the process of inducing the epibranchial placode, a fibroblast growth factor, a bone morphogenetic protein, and a Wnt inhibitor were administered together. Additionally, it was taken into account that it is necessary to additionally suppress the Notch signal for the differentiation of Visceral sensory neurons from the epibranchial placode, and it was also taken into account that beta-nerve growth factor (β-NGF), brain-derived neurotrophic factor (BDNF), and glial cell-derived neurotrophic factor (GDNF) are important for the survival of sensory neurons. Thus, fibroblast growth factor 3 (FGF-3), fibroblast growth factor 10 (FGF-10), bone morphogenetic protein 4 (BMP4), and IWR-1 *(i.e.,* a Wnt inhibitor), DAPT *(i.e.,* a Notch inhibitor), beta-nerve growth factor (β-NGF), brain-derived neurotrophic factor (BDNF), and glial nerve growth factor (GDNF) were used to induce the differentiation of Visceral sensory neurons (FIG. 4a). The nodose ganglion of the vagus nerve, which is one of the representative Visceral sensory neurons, is known to express various chemical-mechanical receptors. Accordingly, immunostaining was performed to confirm whether the induced-Visceral sensory neurons expressed various receptors such as the nodose ganglion (FIG. 4). PHOX2B and NGN2, which are known to be typically expressed in the nodose ganglion, were confirmed to be expressed in the Visceral sensory neurons (FIGS. 4b and 4h). CCKAR, GLP1R, and HTR3A, which are chemoreceptors known to be expressed in the nodose ganglion, were also confirmed to be expressed (FIGS. 4c, 4d, and 4h). TRPV1, which is a nociceptor, was also confirmed to be expressed in the Visceral sensory neurons (FIG. 4e). Additionally, it was confirmed that synaptophysin, which is a presynaptic protein, was expressed in the induced-Visceral sensory neurons (FIG. 4g).

Additionally, in order to confirm whether the expression of induced-Visceral sensory neurons is maintained even at the RNA stage, RT-qPCR was performed on PHOX2B, CCKAR, and TRPV1, which were confirmed by immunostaining, and SSTR1, which is known to be expressed in induced-Visceral sensory neurons. As a result, it was confirmed that all of the above-mentioned factors were successfully expressed in the visceral neurons (FIGS. 5a to 5d).

Additionally, in order to confirm the reproducibility of this protocol, the differentiation of induced-Visceral sensory neurons was performed using three other pluripotent stem cell lines (C71, N33, and CN4 cell lines). As in the previously used pluripotent stem cell line (E3 cell line), it was confirmed that PHOX2B, CCKAR, GLP1R, HTR3A, and TRPV1 were all successfully expressed (FIG. 6).

Through the results above, it was demonstrated that induced-Visceral sensory neurons can be successfully differentiated using the method of the present invention, and that these results show high reproducibility in various cell lines.

In addition to the previous results, in order to confirm whether the same differentiation of Visceral sensory neurons can be observed even when expression of PHOX2B, which is a key genetic factor in the differentiation of Visceral sensory neurons, is induced by injection via lentivirus, a lentivirus that causes PHOX2B-GFP to express at the precursor stage was infected into cells and was differentiated into neurons in the same manner (FIG. 5e). In particular, in a way similar to the previous results, it was confirmed that MAP2 *(i.e.,* a neuronal marker) and GLP1R *(i.e.,* a chemical receptor) were successfully expressed, indicating that a more efficient differentiation of neurons may be possible if lentivirus is used in the future (FIG. 5f).

### Example 3: Demonstrating that induced-Visceral sensory neurons are similar to actual Visceral sensory neurons in terms of function and appearance

In order to confirm the functional aspect of the induced Visceral sensory neurons, the changes in the activity of the neurons were measured using a real-time calcium imaging technique after treating the Visceral sensory neurons with various compounds known to stimulate the same. A total of three compounds *(i.e.,* liraglutide, CCK-8, and capsaicin) were used. Liraglutide corresponds to an agonist of GLP1R and is known to activate the nodose ganglion. It was confirmed that calcium signals were amplified within neurons after liraglutide treatment, thus demonstrating the existence of neurons with functionally effective GLP1R within the induced-Visceral sensory neurons (FIGS. 7a and 7b). CCK-8 is an agonist of the CCK receptor and is known to be able to stimulate the nodose ganglion as well. Likewise, when treated with CCK-8, it was confirmed that the calcium signal was amplified within the cells, thus confirming that neurons including CCK receptors also exist in the induced-Visceral sensory neurons (FIGS. 7c and 7d). Capsaicin is a compound that has excitotoxicity toward TRPV1 and is known to detect pain and heat sensations in the nodose ganglion. Capsaicin also induced the activity of the induced-Visceral sensory neurons, and it was found that the induced-Visceral sensory neurons expressed functionally activated TRPV1 (FIGS. 7e and 7f). Through the results above, it was confirmed that i-VSN expresses functionally effective receptors.

As an analysis of the external aspect, the nerve fiber length and the diameter of the induced-Visceral sensory neurons were directly measured (FIGS. 7g and 7h). The average nerve fiber length was 724.6 µm (SD 333.4), and the average nerve fiber thickness was 1.659 µm (SD1.067). Additionally, the distribution according to nerve fiber thickness showed a bimodal distribution, which is consistent with the results of previous autopsy studies.

### Example 4: Demonstrating that Visceral sensory neurons can be a transporter for the propagation of beta-amyloid and pathological tau protein

In existing animal model studies, it has been reported that the vagus nerve can deliver beta-amyloid and pathological tau protein from the gut to the brain. However, there has been no study as to which nerve between the Visceral sensory neuron component and the visceral motor neuron component of the vagus nerve can play this delivery role. Accordingly, an experiment was performed utilizing a microchip system using a microfluidic device to confirm whether Visceral sensory neurons could serve as a deliverer of pathological proteins. The microfluidic chip system used was the same chip design used in previous studies. This microfluidic chip consists of two chambers and a microfilter connecting the two. Caco-2, which is a colon cancer cell line, was planted in one of the chambers (gut chamber), and in the opposite chamber, an induction-visceral cell line was planted. Epibranchial placode cells, which begin differentiation into sensory neurons, were disassembled into single cells and injected to create the long-subiculum axis (neuron chamber) (FIG. 8a). It was confirmed that within this microfluidic chip environment, the induced Visceral sensory neurons successfully passed through the microfilter and reached the colon cancer cells present in the opposite gut chamber (FIG. 8c).

For the experiments of pathological protein propagation, fluorescent or biotinylated beta-amyloid or tau protein was administered into the gut chamber. In order to prevent these proteins from passing into the neuron chamber due to the diffusion effect, they were blocked using a hydrostatic barrier created by varying the amount of culture medium between the two chambers. In order to confirm the function of this hydrostatic barrier, the tau-441 monomer, which was labeled with a fluorescent material called HiLyte Flour 555 (300 nM), was treated and confirmed whether the color difference was maintained even after 24 hours (FIGS. 9a and 9b). By examining the gut chamber 48 hours after treatment with biotin-labeled tau-441 monomer (300 nM), it was confirmed that the biotin signal overlapped with the neurite signal of the induced-Visceral sensory neurons that had passed through the microfilter (FIG. 8d). These signals were also observed within the induced-visceral neurons present in the opposite neuron chamber after crossing the fine filter, suggesting that the tau protein uptaken into the neurites of the induced-Visceral sensory neurons in the gut chamber passed to the opposite neuron chamber (FIG. 8e). Additionally, in order to exclude the possibility that it was an endogenous biotin signal, tau fibrils labeled with ATTO488 fluorescence were also treated. In particular, it was confirmed that the ATTO488 fluorescence signal also overlapped with the neurite signal of the induced-Visceral sensory neurons in immunostaining, which is a result suggesting that the previously seen signal was not an endogenous biotin signal but a signal of biotin attached to tau (FIG. 9c)). Next, beta-amyloid (1-42) (3.6 µM) labeled with HiLyte Flour 647 fluorescent was also treated in the gut chamber. The corresponding fluorescence signals were also observed within the neurite signals of the induced-Visceral sensory neurons in both the gut chamber and the neuron chamber, thus suggesting that the induced-Visceral sensory neurons are also involved in the propagation of beta-amyloid (FIGS. 8f and 8g). It is known that tau and beta-amyloid can appear in both axons and dendrites. Visceral sensory neurons are pseudounipolar neurons and have long dendrites similar to axons. Therefore, both axons and dendrites can arrive at the gut chamber. This phenomenon can explain that beta-amyloid and tau passed into the neuron chamber through axons or dendrites.

In Alzheimer's disease, it is known that APOE4 expressed in neurons can promote the propagation of tau and beta-amyloid. Accordingly, the present inventors made an attempt to determine how the APOE genotype affects the uptake of beta-amyloid and tau protein in induced-Visceral sensory neurons. Accordingly, two genetically modified pluripotent stem cell lines, E3 and E4 cell lines, were used for one test subject. Quantitative analysis of propagation was performed on induced-Visceral sensory neurons derived from the two types of cell lines in a microchip environment. Biotinylated tau-441 (15nM) protein or FITC-labeled beta-amyloid oligomer (1uM) were each treated and the results were confirmed 72 hours later. Among these, it was confirmed that the E4 cell line having the APOE4/4 genotype showed a statistically significant increase in the amount of biotinylated tau and FITC-labeled beta-amyloid oligomers (FIGS. 8h to 8m). Through the foregoing, it was confirmed that APOE4 is a gene that can significantly affect the propagation of beta-amyloid and tau protein through Visceral sensory neurons.

### References

1. Cryan, J. F., O'Riordan, K. J., Sandhu, K., Peterson, V. & Dinan, T. G. The gut microbiome in neurological disorders. Lancet Neurol 19, 179-194 (2020).
2. Kim, M. S. et al. Transfer of a healthy microbiota reduces amyloid and tau pathology in an Alzheimer's disease animal model. Gut 69, 283-294 (2020).
3. Choi, H., Lee, D. & Mook-Jung, I. Gut Microbiota as a Hidden Player in the Pathogenesis of Alzheimer's Disease. J Alzheimers Dis 86, 1501-1526 (2022).
4. Vargas-Caballero, M. et al. Vagus nerve stimulation as a potential therapy in Early Alzheimer's disease: a review. Frontiers in human neuroscience 16 (2022).
5. Kim, S. et al. Transneuronal Propagation of Pathologic α-Synuclein from the Gut to the Brain Models Parkinson's Disease. Neuron 103, 627-641.e627 (2019).
6. Chen, C. et al. Gut inflammation triggers C/EBPβ/δ-secretase-dependent gut-to-brain propagation of Aβ and Tau fibrils in Alzheimer's disease. Embo j 40, e106320 (2021).
7. Sun, Y. et al. Intra-gastrointestinal amyloid-beta1-42 oligomers perturb enteric function and induce Alzheimer's disease pathology. J Physiol 598, 4209-4223 (2020).
8. Crespo, M. et al. Colonic organoids derived from human induced pluripotent stem cells for modeling colorectal cancer and drug testing. Nat Med 23, 878-884 (2017).
9. Pasca, A. M. et al. Functional cortical neurons and astrocytes from human pluripotent stem cells in 3D culture. Nat Methods 12, 671-678 (2015).
10. De Santis, R. et al. Direct conversion of human pluripotent stem cells into cranial motor neurons using a piggyBac vector. Stem Cell Res 29, 189-196 (2018).
11. Schwartzentruber, J. et al. Molecular and functional variation in iPSC-derived sensory neurons. Nat Genet 50, 54-61 (2018).
12. Kupari, J., Haring, M., Agirre, E., Castelo-Branco, G. & Ernfors, P. An Atlas of Vagal Sensory Neurons and Their Molecular Specialization. Cell Rep 27, 2508-2523.e2504 (2019).
13. Saint-Jeannet, J. P. & Moody, S. A. Establishing the pre-placodal region and breaking it into placodes with distinct identities. Dev Biol 389, 13-27 (2014). 14. Ladher, R. K., O'Neill, P. & Begbie, J. From shared lineage to distinct functions: the development of the inner ear and epibranchial placodes. Development 137, 1777-1785 (2010).
15. Koehler, K. R. et al. Generation of inner ear organoids including functional hair cells from human pluripotent stem cells. Nat Biotechnol 35, 583-589 (2017).
16. McCarroll, M. N. & Nechiporuk, A. V. Fgf3 and Fgf10a work in concert to promote maturation of the epibranchial placodes in zebrafish. PLoS One 8, e85087 (2013).
17. Vermeiren, S., Bellefroid, E. J. & Desiderio, S. Vertebrate Sensory Ganglia: Common and Divergent Features of the Transcriptional Programs Generating Their Functional Specialization. Front Cell Dev Biol 8, 587699 (2020).
18. Blentic, A., Chambers, D., Skinner, A., Begbie, J. & Graham, A. The formation of the cranial ganglia by placodallyderived sensory neuronal precursors. Mol Cell Neurosci 46, 452-459 (2011).
19. Wang, L. et al. Notch signalling regulates epibranchial placode patterning and segregation. Development 147 (2020).
20. Katz, D. M., Erb, M., Lillis, R. & Neet, K. Trophic regulation of nodose ganglion cell development: evidence for an expanded role of nerve growth factor during embryogenesis in the rat. Exp Neurol 110, 1-10 (1990).
21. Erickson, J. T., Brosenitsch, T. A. & Katz, D. M. Brain-derived neurotrophic factor and glial cell line-derived neurotrophic factor are required simultaneously for survival of dopaminergic primary sensory neurons in vivo. J Neurosci 21, 581-589 (2001).
22. Borgmann, D. et al. Gut-brain communication by distinct sensory neurons differently controls feeding and glucose metabolism. Cell Metab 33, 1466-1482 e1467 (2021).
23. Adams, J. M. et al. Liraglutide Modulates Appetite and Body Weight Through Glucagon-Like Peptide 1 Receptor-Expressing Glutamatergic Neurons. Diabetes 67, 1538-1548 (2018).
24. Browning, K. N., Babic, T., Holmes, G. M., Swartz, E. & Travagli, R. A. A critical re-evaluation of the specificity of action of perivagal capsaicin. J Physiol 591, 1563-1580 (2013).
25. Havton, L. A. et al. Human organ donor-derived vagus nerve biopsies allow for well-preserved ultrastructure and high-resolution mapping of myelinated and unmyelinated fibers. Sci Rep 11, 23831 (2021).
26. Kim, C. et al. HDAC6 inhibitor blocks amyloid betainduced impairment of mitochondrial transport in hippocampal neurons. PLoS One 7, e42983 (2012).
27. Takeda, S. et al. Neuronal uptake and propagation of a rare phosphorylated high-molecular-weight tau derived from Alzheimer's disease brain. Nat Commun 6, 8490 (2015).
28. Takeda, S. Tau Propagation as a Diagnostic and Therapeutic Target for Dementia: Potentials and Unanswered Questions. Front Neurosci 13, 1274 (2019).
29. Nath, S. et al. Spreading of neurodegenerative pathology via neuron-to-neuron transmission of beta-amyloid. J Neurosci 32, 8767-8777 (2012).
30. Goaillard, J. M., Moubarak, E., Tapia, M. & Tell, F. Diversity of Axonal and Dendritic Contributions to Neuronal Output. Front Cell Neurosci 13, 570 (2019).
31. Koutsodendris, N. et al. Neuronal APOE4 removal protects against tau-mediated gliosis, neurodegeneration and myelin deficits. Nat Aging 3, 275-296 (2023).
32. Kim, H., Kim, S., Cho, B., Shin, J. & Kim, J. APOE epsilon4-dependent effects on the early amyloid pathology in induced neurons of patients with Alzheimer's disease. Transl Neurodegener 11, 45 (2022).
33. Park, J. C. et al. A logical network-based drugscreening platform for Alzheimer's disease representing pathological features of human brain organoids. Nat Commun 12, 280 (2021).
34. Shin, W. & Kim, H. J. 3D in vitro morphogenesis of human intestinal epithelium in a gut-on-a-chip or a hybrid chip with a cell culture insert. Nat Protoc 17, 910-939 (2022).

## Claims

1. A method for differentiating Visceral sensory neurons from induced pluripotent stem cells, the method comprising:
(a) inducing an otic-epibranchial progenitor domain from induced pluripotent stem cells;
(b) culturing the otic-epibranchial progenitor domain to induce epibranchial placode; and
(c) differentiating the epibranchial placode into Visceral sensory neurons.

2. The method of claim 1, wherein the culturing in the step (b) is performed in the presence of FGF-3, FGF-10, BMP-4, and IWR-1.

3. The method of claim 1, wherein the differentiating in the step (c) is performed in the presence of FGF-3, FGF-10, BMP-4, IWR-1, DAPT, BDNF, GDNF, and NGF-β.

4. The method of claim 1, wherein in the Visceral sensory neurons differentiated by the method above, one or more factors selected from the group consisting of PHOX2B, NGN2, CCKAR, GLP1R, HTR3A, TRPV1, synaptophysin, SSTR1, and MAP2 are expressed.

5. The method of claim 1, wherein the Visceral sensory neurons differentiated by the method above deliver beta-amyloid or tau protein from the gut to the brain.

6. A composition for inducing differentiation of Visceral sensory neurons from induced pluripotent stem cells, comprising FGF-3, FGF-10, BMP-4, and IWR-1.

7. The composition of claim 6, wherein the composition further comprises DAPT, BDNF, GDNF, and NGF-β.
